# EUROPÄISCHE PATENTSCHRIFT

(11) **EP 0 975 287 B1**
(45) Veröffentlichungstag und Bekanntmachung des Hinweises auf die Patenterteilung: **01.09.2004**
(21) Anmeldenummer: 98924169.0
(22) Anmeldetag: 22.04.1998
(51) Int. Cl.: A61F 2/38

(54) **FEMURSCHLITTEN**
FEMORAL SLED PROSTHESIS
PROTHESE FEMORALE A GLISSEMENT UNICOMPARTIMENTALE

(30) Priorität: 22.04.1997 DE 19716879
(43) Veröffentlichungstag der Anmeldung: 02.02.2000
(73) Patentinhaber: PLUS Endoprothetik AG, 6343 Rotkreuz (CH)
(72) Erfinder: SCHMOTZER, Hans, CH-5000 Aarau (CH); SCHULER, Peter, D-76228 Karlsruhe (DE); MALZER, Udo, D-76185 Karlsruhe (DE)
(74) Vertreter: Popp, Eugen, Dr.
(86) Internationale Anmeldenummer: PCT/EP1998/002376
(87) Internationale Veröffentlichungsnummer: WO 1998/047448

(56) Entgegenhaltungen:
- US-A- 4 888 020
- US-A- 5 326 361
- US-A- 5 549 688

## Beschreibung

Die Erfindung betrifft einen Femurschlitten mit zwei konvex gewölbten Kondylenschalen, die anterior durch ein Patellaschild starr miteinander verbunden sind.

Derartige Femurschlitten sind allgemein bekannt. Es wird diesbezüglich nur beispielhaft verwiesen auf die DE 40 41 920 C2 oder WO 87/02882.

In der heute beim Kniegelenkersatz üblichen Operationstechnik am Femur wird das Implantat an den hinteren bzw. posterioren Kondylen ausgerichtet. Von beiden Kondylen wird gleich viel Knochen entfernt. Der anteriore oder ventrale Schnitt ist parallel zum posterioren bzw. dorsalen Schnitt und damit zum posterioren Kondylenrand. Dies hat jedoch aus verschiedenen technischen und anatomischen Gründen den Nachteil, daß erstens die Rotationsachse nicht mehr mit der durch die Seitenbänder vorgegebenen Achse übereinstimmt und zweitens das Implantat durch die nichtanatomische Position in der Beugung entweder auf der medialen Seite zu eng oder auf der lateralen Seite lose sitzt.

Aus diesem Grund wird von Experten in der Kniegelenk-Endoprothetik eine Außenrotation für die Kondyle empfohlen. Für die Orientierung dient entweder die sogenannte Epikondylenachse oder die sogenannte "Whiteside"-Linie. Durch diese Außenrotation der Schnittlehre wird im posterioren Bereich lateral weniger Knochen relativ zur medialen Seite entfernt. Gleichzeitig wird anterio-lateral mehr Knochen verglichen mit der medialen Seite reseziert. Da jedoch die Implantate auf die ursprüngliche Schnittführung abgestimmt sind, die gekennzeichnet ist durch gleiche Materialdicke medial und lateral, ergibt sich ein höherer Aufbau auf der lateralen Seite, welcher das unerwünschte Spiel auf der lateralen Seite in der Beugung kompensiert. Gleichzeitig wird durch die Außenrotation des Implantats die Patellagrube nach lateral versetzt, was die Gefahr von Patella-Subluxationen reduziert. Dieses Verfahren hat jedoch die folgenden Nachteile:
- Der anterio-laterale Rand des Implantats steht häufig über den Knochenrand vor.
- Aufgrund der tieferen Schnittführung auf der lateralen Seite wird häufig die anteriore Cortex angeschnitten, was aus verschiedenen Gründen nicht wünschenswert ist.
- Auf der medialen Seite steht das Patellaschild des Implantats über den Knochen vor, d. h. es verbleibt ein Spalt zwischen Schild und Knochen. Der Grund dafür ist die natürliche Geometrie des anterioren Femurs, welcher im distalen Bereich nach medial geneigt ist.

In der US 5 549 688 ist ein asymmetrischer Femurschlitten gemäß der Merkmalen des Oberbegriffs des Anspruchs 1 offenbart, der eine dünnere posterior-mediale Kondyle und eine dickere posterior-laterale Kondyle aufweist und somit den Sägeschnitt am Knochen vereinfachen soll.

Sowohl der letztgenannte Femurschlitten gemäß der US 5 549 688, als auch die eingangs genannten Femurschlitten gemäß der DE 40 41 920 C2 oder WO 87/02882 besitzen den Nachteil, daß sich der Femurschlitten bei einer Einwirkung von Querkraft in mediale Richtung vom Knochen lösen kann, da nicht für eine ausreichende Fixierung des Implantats am Knochen für den Fall medial gerichteter Querkräfte gesorgt ist.

Der vorliegenden Erfindung liegt die Aufgabe zugrunde, einen Femurschlitten zur Verfügung zu stellen, der medial gerichtete Querkräfte besser aufnehmen kann, wobei die Sägeschnitte am distalen Ende des Femurs bestmöglich an dessen Anatomie angepaßt sind.

Diese Aufgabe wird durch die kennzeichnenden Merkmale des Anspruches 1 gelöst, wobei bevorzugte konstruktive Details und Abwandlungen des erfindungsgemäßen Grundkonzepts in den Unteransprüchen angegeben sind.

Der Kern der vorliegenden Erfindung liegt also darin, daß die anterior-lateralen und und anterior-medialen Paßflächen sich parallel zueinander, insbesondere auch parallel zur Bezugsebene erstrecken. Somit werden Zugspannungen beim Einwirken medial gerichteter Querkräfte verhindert. Zusätzlich ist durch die erfindungsgemäße Konstruktion eine bessere Anpassung der Schnitte an die Anatomie des Patienten gewährleistet, als dies bei den Implantaten gemäß dem Stand der Technik der Fall ist.

Nachstehend wird eine Ausführungsform eines erfindungsgemäß ausgebildeten Femurschlittens anhand der beigefügten Zeichnung näher erläutert. Es zeigen:
- Figur 1: einen Femurschlitten gemäß Erfindung in perspektivischer Ansicht von schräg oben;

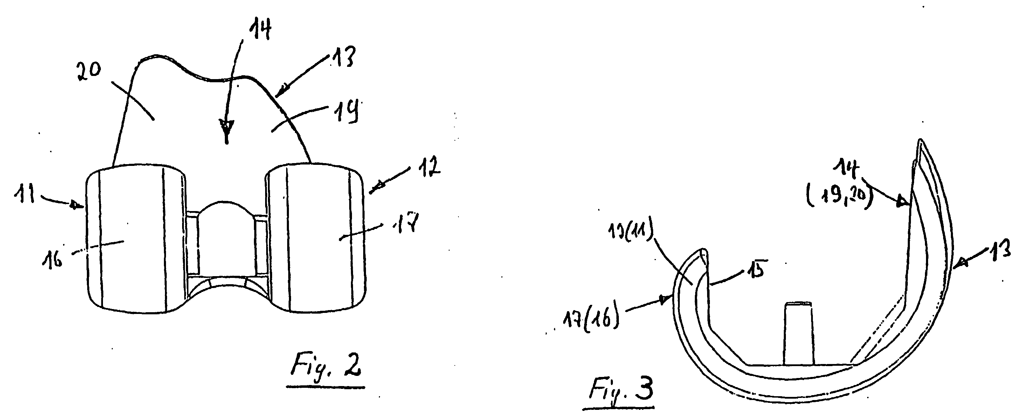

- Figur 2: den Femurschlitten gemäß den Figuren 1 und 2 in Ansicht von hinten bzw. posterior; und
- Figur 3: den Femurschlitten gemäß den vorangehenden Figuren in Seitenansicht, und zwar in Ansicht von medial.

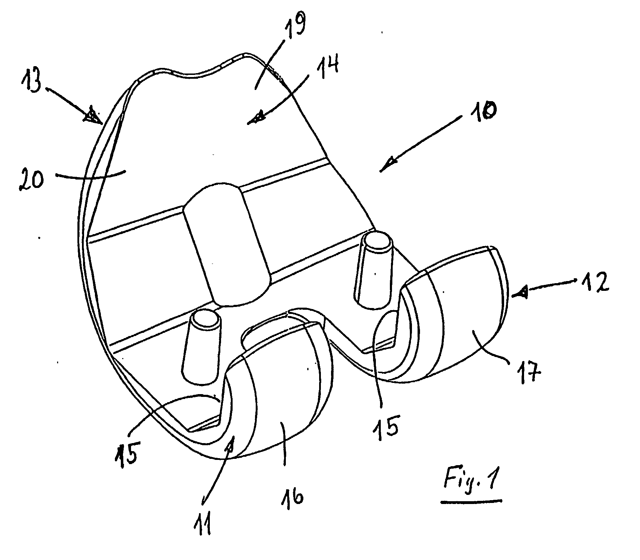

In Figur 1 ist ein Femurschlitten 10 dargestellt, der gekennzeichnet ist durch zwei konvex gewölbte Kondylenschalen 11, 12 sowie durch ein Patellaschild 13. Durch das Patellaschild 13 sind die beiden Kondylenschalen 11, 12 starr miteinander verbunden. Insoweit handelt es sich um eine an sich bekannte Konstruktion eines Femurschlittens. Die beiden Kondylenschalen 11, 12 und das Patellaschild 13 definieren innenseitig einem femuralen Ventralschnitt einerseits und femuralen Dorsalschnitt andererseits entsprechende anteriore und posteriore Paßflächen 14, 15. Diese Paßflächen sind der ventralen und dorsalen Sägeschnittfläche am distalen Ende des Femurs zugeordnet.

Die posterioren Gleitflächen der Kondylenschalen 11, 12, die gleitkufenartig ausgebildet sind, sind mit den Bezugsziffern 16 und 17 gekennzeichnet. Die posterioren Gleitflächen 16, 17 der Kondylenschalen 11, 12 definieren eine Bezugsebene. In bezug auf diese Bezugsebene liegt die anterio-mediale Paßfläche 19 dieser Bezugsebene näher als die anterio-laterale Paßfläche 20. Im konkreten Fall erstrecken sich die anterio-laterale und anterio-mediale Paßfläche etwa parallel zueinander, insbesondere auch parallel zur Bezugsebene.

Die Verbindungsfläche zwischen der anterio-lateralen und anterio-medialen Paßfläche kann durchgehend eben oder alternativ gewölbt bzw. geschwungen ausgebildet sein. Bei der dargestellten Ausführungsform handelt es sich um eine ebene Fläche, die integral übergeht in die beiden Paßflächen 19, 20.

Die erwähnten Ausführungsformen sind patientenabhängig. Sie zeichnen sich jedoch allesamt durch die eingangs genannte Zielsetzung aus.

Die posterio-laterale und posterio-mediale Paßflächen 15 erstrecken sich bei der dargestellten Ausführungsform parallel zur Bezugsebene, wobei der Abstand zur Bezugsebene jeweils der gleiche ist.

Es ist auch denkbar, daß der Abstand der beiden posterioren Paßflächen 15 von der Bezugsebene unterschiedlich ist. Des weiteren ist es denkbar, daß sich diese Paßflächen unter einem Winkel, und zwar entweder gemeinsamen oder unterschiedlichen Winkel zur Bezugsebene erstrecken. Schließlich ist auch denkbar, daß sich die posterioren Paßflächen 15 parallel zu den anterioren Paßflächen erstrecken.

### Bezugszeichenliste

- 10: Femurschlitten
- 11: Kondylenschale
- 12: Kondylenschale
- 13: Patellaschild
- 14: anteriore Paßfläche
- 15: posteriore Paßfläche
- 16: posteriore Gleitfläche
- 17: posteriore Gleitfläche
- 19: anterio-mediale Paßfläche
- 20: anterio-laterale Paßfläche

## Patentansprüche

1. Femurschlitten mit zwei konvex gewölbten Kondylenschalen (11, 12), die anterior durch ein Patellaschild (13) starr miteinander verbunden sind, wobei die beiden Kondylenschalen (11, 12) und das Patellaschild (13) innenseitig einem femuralen Ventralschnitt einerseits und femuralen Dorsalschnitt andererseits entsprechende anteriore (14; 19, 20) und posteriore (15) Paßflächen definieren, wobei
in Bezug auf eine durch die posterioren Gleitflächen (16, 17) der Kondylenschalen (11, 12) definierte Ebene die anterior-mediale Paßfläche (19) dieser Bezugsebene näher liegt als die anterior-laterale Paßfläche (20);
**dadurch gekennzeichnet, daß**
die anterior-lateralen und anterior-medialen Paßflächen sich parallel zueinander, insbesondere auch parallel zur Bezugsebene erstrecken.

2. Femurschlitten nach Anspruch 1,
**dadurch gekennzeichnet, daß**
die Verbindungsfläche zwischen der anterior-lateralen und anterior-medialen Paßfläche (19, 20) eine durchgehend ebene oder alternativ gewölbte Übergangsfläche ist.

3. Femurschlitten nach Anspruch 1 oder 2,
**dadurch gekennzeichnet, daß**
die posterioren, d. h. die posterior-laterale Paßfläche (15) und posterior-mediale Paßfläche (15) sich entweder parallel oder unter einem Winkel, insbesondere entsprechend der Neigung der anterioren Paßfiäche(n) (14; 19, 20), zur Bezugsebene erstrecken.

4. Femurschlitten nach einem der Ansprüche 1 bis 5,
**dadurch gekennzeichnet, daß**
die posterioren Paßflächen (15) lateral und medial von der Bezugsebene entweder gleich oder unterschiedlich beabstandet sind.

## Claims

1. A femoral component comprising two convexly curved condylar shells (11, 12) rigidly connected to one another in the anterior direction by a patellar shield (13), wherein the two condylar shells (11, 12) and the patellar shield (13) define, on their inner surfaces, anterior (14; 19, 20) and posterior (15) locating surfaces corresponding to a ventral femoral cut on one side and a dorsal femoral cut on the other side, wherein, in relation to a plane defined by the posterior sliding surfaces (16, 17) of the condylar shells (11, 12), the anterior-medial locating surface (19) lies closer to this reference plane than the anterior-lateral locating surface (20), **characterised in that** the anterior-lateral and anterior-medial locating surfaces extend parallel to one another, in particular also parallel to the reference plane.

2. A femoral component according to claim 1, **characterised in that** the connecting surface between the anterior-lateral and anterior-medial locating surface (19, 20) is a continuous flat or, alternatively, curved transition surface.

3. A femoral component according to claim 1 or 2, **characterised in that** the posterior locating surfaces, i.e. the posterior-lateral locating surface (15) and posterior-medial locating surface (15), extend either parallel or at an angle to the reference plane, in particular at an angle corresponding to the inclination of the anterior locating surface(s) (14; 19, 20).

4. A femoral component according to any one of claims 1 to 5 [sic], **characterised in that** the lateral and medial posterior locating surfaces (15) are either uniformly or differently spaced from the reference plane.

## Revendications

1. Prothèse fémorale à glissement comportant deux coquilles condylaires (11, 12) convexes qui sont reliées rigidement entre elles sur le côté antérieur par une plaque de rotule (13), les deux coquilles condylaires (11, 12) et la plaque de rotule (13) définissant côté intérieur, des surfaces d'ajustement antérieures (14; 19, 20) et postérieures (15) correspondant d'une part à une coupe ventrale fémorale et d'autre part à une coupe dorsale fémorale, la surface d'ajustement médiale antérieure (19) se situant, par rapport à un plan défini par les surfaces de glissement postérieures (16, 17) des coquilles condylaires (11, 12), plus près de ce plan de référence que la surface d'ajustement latérale antérieure (20) ;
**caractérisée en ce que**
les surfaces d'ajustement latérales antérieures et médiales antérieures s'étendent parallèlement les unes aux autres, en particulier parallèlement aussi au plan de référence.

2. Prothèse fémorale à glissement selon la revendication 1,
**caractérisée en ce que**
la surface de liaison entre la surface d'ajustement latérale antérieure et la surface d'ajustement médiale antérieure (19, 20) est une surface de transition plane continue ou alternativement bombée.

3. Prothèse fémorale à glissement selon la revendication 1 ou 2,
**caractérisée en ce que**
les surfaces d'ajustement postérieures, c'est-à-dire la surface d'ajustement latérale postérieure (15) et la surface d'ajustement médiale postérieure (15) s'étendent soit parallèlement, soit suivant un angle, en particulier en fonction de l'inclinaison de la (des) surface(s) d'ajustement antérieure(s) (14 ; 19, 20), par rapport au plan de référence.

4. Prothèse fémorale à glissement selon l'une des revendications 1 à 5,
**caractérisée en ce que**
les surfaces d'ajustement postérieures (15) sont espacées latéralement et médialement du plan de référence, soit d'une même distance, soit d'une distance différente.
